# EUROPEAN PATENT APPLICATION

(11) **EP 1 552 739 A1**
(43) Date of publication of application: **13.07.2005**
(21) Application number: 03741384.6
(22) Date of filing: 14.07.2003
(51) Int. Cl.: A01K 67/027

(54) **HEPATIC CIRRHOSIS MODEL ANIMAL AND METHOD OF CONSTRUCTING THE SAME**

(30) Priority: 16.07.2002 JP 2002207442
(71) Applicant: Japan Science and Technology Agency, Kawaguchi-shi, Saitama 332-0012 (JP)
(72) Inventor: KURODA, Shunichi, Suita-shi, Osaka 565-0872 (JP); TANIZAWA, Katsuyuki, Toyono-gun, Osaka 563-0214 (JP); KONDO, Akihiko, Kobe-shi, Hyogo 657-0015 (JP); UEDA, Masakazu, Shinjuku-ku, Tokyo 162-0837 (JP); SENO, Masaharu, Okayama-shi, Okayama 703-8273 (JP)
(74) Representative: Bentz, Jean-Paul
(86) International application number: PCT/JP2003/008939
(87) International publication number: WO 2004/006663

(57) **Abstract**

The present invention provides a cirrhosis model animal having human hepatic tissues affected with cirrhosis. Anti-asialo GM1 antibodies are administered to a scid mouse which is an immune-deficiency animal, and its natural-killer-cell-dependent immune response capability is made defective, and then cirrhosis-patient-derived hepatic tissues are transplanted beneath a kidney membrane of the scid mouse, thereby producing the cirrhosis model animal. The cirrhosis model animal has the human hepatic tissues affected with cirrhosis, so that it is possible to use the cirrhosis model animal in development of a therapy and a therapeutic drug for cirrhosis.

## Description

### TECHNICAL FIELD

The present invention relates to a cirrhosis model animal and a production method thereof. The invention particularly relates to a cirrhosis model animal having human hepatic tissues affected with cirrhosis and a production method thereof.

### BACKGROUND ART

Cirrhosis is such a hepatic diffuse disease that continual necrosis of hepatic cells causes a liver to have fibrillary hyperplasia and varicose regeneration of remaining hepatic cells. The disease results from infection (hepatitis virus, parasite), alcohol, bile congestion, hepatic congestion, nutritional disorder, intoxication, metabolic disorder, autoimmunity, and the like. It is often that each of these factors causes chronic hepatitis to be cirrhosis.

Cirrhosis is an irreversible hepatic degenerative disease and causes various hepatic dysfunctions. Further, it is often that cirrhosis results in a hepatic cancer, which is highly likely to results in death. Thus, an effective therapy thereof is desired. At the present, although there are so many patients that cirrhosis is called "folk disease", there is only an experimental example showing that administration of a hepatic regenerating factor HGF (referred to also as "hepatic cell growth factor") in a gene therapy improves the symptom, and there is no reliable therapy for cirrhosis.

Incidentally, an animal experiment using a model animal affected with a disease whose pathology is extremely similar to pathology of human disease plays an important role in resolving how the human disease is developed and in developing a therapy and a therapeutic drug for the disease. Also with respect to cirrhosis, the model animal is used to resolve how cirrhosis is developed and to develop a therapy and a therapeutic drug for cirrhosis.

A cirrhosis model animal currently used is produced by orally administering carbon tetrachloride to a mouse, a rat, and the like, and breaking down hepatic tissues thereof. The model animal is suitable for observation on how hepatic tissues are regenerated, but does not have genuine cirrhosis tissues. This is a problem in developing the therapy and the therapeutic drug for cirrhosis.

The present invention was made in view of the foregoing problems, and an object of the invention is to provide a cirrhosis model animal having hepatic tissues actually affected with cirrhosis and a production method thereof.

### DISCLOSURE OF INVENTION

In view of the foregoing problems, the inventors of the present invention diligently studied a model animal having cirrhosis tissues and a production method thereof. As a result, they succeeded in efficiently suppressing a natural-killer-cell-dependent rejection by administering anti-asialo GMI antibodies to an immune-deficiency mouse (scid mouse) in advance. Further, they found it possible to transplant cirrhosis-patient-derived human hepatic tissues beneath a hepatic membrane of the immune-deficiency mouse and to maintain the human hepatic tissues over an extended period of time, thereby completing the present invention.

A cirrhosis model animal of the present invention is characterized in that a human hepatic tissue affected with cirrhosis is transplanted in a tissue of an animal.

Thus, the hepatic tissue affected with cirrhosis is engrafted into the animal. As used herein, "the hepatic tissue is engrafted" means that the transplanted hepatic tissue functions in a body without necrotizing, and the cirrhosis model animal in which the hepatic tissue has been transplanted survives.

Thus obtained cirrhosis model animal is a novel model animal that has not ever been produced. Thus, unlike a conventional model animal whose hepatic tissue is damaged, the cirrhosis model animal according to the present invention has the human hepatic tissue actually affected with cirrhosis, so that it is possible to favorably use the cirrhosis model animal in: pathology analysis of cirrhosis; development of a therapeutic drug; and improvement of a therapy. Further, it is possible to prevent cirrhosis from proceeding to hepatic cancer, so that this technique plays an important role in preventing these diseases.

The animal tissue in which the hepatic tissue is transplanted is not particularly limited, but it is preferable to use a tissue which maintains a high blood flow so that the hepatic tissue is not necrotized by ischaemia. Examples of such tissue include a kidney, a liver, a spleen, a muscle, and the like. Among them, it is particularly preferable that the hepatic tissue is transplanted in the animal kidney, more specifically, beneath a kidney membrane thereof. When the hepatic tissue is transplanted in the kidney in this manner, it is possible to engraft the hepatic tissue in a relatively brief period (approximately one week). Further, it is possible to easily distinguish the kidney from the transplanted tissues. Furthermore, it is possible to transplant the tissues separately into a right portion, a left portion, or an upper portion, a middle portion, and a lower portion of the kidney of a single organism. Thus, this results in such advantage that it is possible to carry out comparison in terms of drug efficacy under the same condition.

In the present invention, it is preferable that the animal is an immune-deficiency animal.

As used herein, "immune deficiency" refers to a condition under which: a portion or some portions of cell components constituting an immune system are defective or dysfunction, so that a normal immune mechanism is damaged. In other words, "immune deficiency" means a condition under which: congenital immunity and/or acquired immunity are suppressed so that the transplanted hepatic tissues are engrafted into an animal. Note that, it can be said that the immune-deficiency animal is an immunocompromised animal.

That is, it is preferable to arrange the immune deficiency animal so that immunocompetent cells or factors involved in immune response are partially or entirely defective. Specifically, it is preferable that the immune deficiency animal is an animal whose T-cell and/or B-cell-dependent immune response capability is defective. Further, it is more preferable that the immune-deficiency animal is an animal whose natural-killer-cell (NK cell) dependent immune response capability is defective (or the immune response capability is suppressed). When many immunocompetent cells or many factors involved in immune response are defective, it may be possible to suppress the immune response dependent on the cells or the factors, so that it may be possible to depress rejection at the time of transplantation of the hepatic tissues.

Therefore, examples of the immune deficiency animal include: a nude animal whose T-cell-dependent immune response capability is defective since the nude animal has no thymus; a scid animal whose B-cell-dependent immune response capability is defective as well as the immune response capability of the nude animal; an animal whose NK-cell-dependent immune response capability is defective as well as the immune response capability of the scid animal.

As the foregoing immune deficiency animal, it is possible to produce a mouse whose T-cell, B-cell, and NK-cell-dependent immune response capability is made defective by administering anti-asialo GM1 antibodies to a generally used scid mouse. Asialo GM1 is a protein specifically expressed in NK cells. Therefore, when the anti-asialo GM 1 antibodies are administered to the scid mouse, not only the T-cell and B-cell-dependent immune response capability but also the NK-cell-dependent immune response capability can be made defective.

In the present invention, the animal is not particularly limited, but it is possible to use a mouse, a rat, a guinea pig, a hamster, a rabbit, a dog, and the like. Among them, it is preferable to use the mouse, the rat, and the rabbit, as an experimental animal, and it is particularly preferable to use the mouse since tissues can be engrafted therein in a relatively brief period (approximately one week) and the mouse can be obtained at low cost.

Further, in the cirrhosis model animal of the present invention, it is preferable that the human hepatic tissue affected with the cirrhosis is classified as Child A in accordance with Child's classification which classifies cirrhosis in terms of severity.

That is, it is preferable that the human hepatic tissue transplanted in order to obtain the cirrhosis model animal is slightly affected with cirrhosis, and it is difficult to transplant a severe cirrhosis tissue which can be classified as Child C. When the hepatic tissue is so slightly affected with cirrhosis that its condition is classified as Child A in accordance with Child's classification, it is possible to improve a rate at which the hepatic tissue is engrafted into the cirrhosis model animal.

It is possible to produce the cirrhosis model animal according to the present invention by transplanting a hepatic tissue affected with cirrhosis in a tissue of an immune-deficiency animal whose immune response capability has been made defective, preferably beneath a kidney membrane of the immune-deficiency animal, and by engrafting the transplanted hepatic tissue.

Thus, it is possible to engraft the cirrhosis tissue into the model animal with a high probability, and it is possible to maintain the cirrhosis tissue over an extended period of time. Thus, it is possible to easily produce the cirrhosis model animal which is useful in development of a therapy and a therapeutic drug for cirrhosis.

### BEST MODE FOR CARRYING OUT THE INVENTION

The following description will explain an embodiment of the present invention.

(1) A cirrhosis model animal of the present invention and a method of the present invention for producing the cirrhosis model animal

The cirrhosis model animal according to the present invention is produced by transplanting human hepatic tissues affected with cirrhosis into a body of an animal and engrafting the human hepatic tissues into the body. That is, the cirrhosis model animal is produced by transplanting human hepatic tissues, extirpated from a patient affected with cirrhosis, into other animal, and engrafting the human hepatic tissues into a body of the animal.

The "human hepatic tissues" used as a sample can be easily obtained since hepatectomy is carried out even in a general hospital as a therapy for hepatic cancer and there are many patients affected with the hepatic cancer. As the "human hepatic tissues", it is preferable to use tissues slightly affected with cirrhosis. According to Child's classification which classifies cirrhosis in terms of severity, it is preferable to use Child-A hepatic tissues as the "human hepatic tissues". Note that, it may be difficult to transplant Child-C hepatic tissues. Further, the transplanted hepatic tissues may be normal hepatic tissues derived from a person who is not affected with cirrhosis. In this case, it may be so arranged that: a model animal is produced by transplanting normal hepatic tissues into tissues of an animal described later, and then the normal hepatic tissues transplanted into the model animal are caused to be affected with cirrhosis.

Further, the "human hepatic tissues" may be prepared by transplanting a tissue piece from which hepatic tissues affected with cirrhosis have been removed, or may be prepared by culturing the tissue piece in the same organism or other organism (culture in vivo) so that the tissue piece is subcultured, or may be prepared by culturing the tissue piece in a glass vessel (culture in vitro).

Note that, it is preferable that the "human hepatic tissues" are conserved in ice-cold culture solution (such as cell culture solution, physiological saline, and the like) after being extirpated from a cirrhosis patient. Further, although depending on a size of an organism in which the tissues are transplanted, it is preferable to cut the transplanted hepatic tissues into an appropriate size such as an approximately 2 mm cube in case of transplanting the tissues into a mouse and a rat for example.

The animal tissues in which the sample is transplanted is not particularly limited as long as the sample can be engrafted, but it is preferable to use tissues which maintain a high blood flow so that the transplanted hepatic tissues are not necrotized by ischaemia. Examples of such tissues include a kidney, a liver, a spleen, a muscle, and the like. Among them, it is particularly preferable to transplant the hepatic tissues in the animal kidney, more specifically, beneath a kidney membrane thereof. "to transplant the hepatic tissues beneath a kidney membrane" means that a membrane of a kidney in which the hepatic tissues are to be transplanted is cut open and the hepatic tissues are inserted into the cut-open portion.

Further, the animal tissues may be tissues, such as a bulbar conjunctiva, which hardly reject the sample.

In this manner, not only the technique of transplanting the sample in the animal tissues which hardly reject the sample but also the technique of using an immune-deficiency animal whose immune response has been made defective prevents the animal tissues from rejecting the sample.

The immune-deficiency animal means an animal which is in an immune deficiency state because of (i) disorder of immunocompetent cells, (ii) disorder of genes such as immunoresponse genes, (iii) physicochemical factors such as administration of a drug e.g., an immune-suppressing drug and the like or irradiation of an x-ray, (iv) and the like. Such an immune-deficiency animal can be obtained by mutation or artificial production.

A method for artificially producing the immune-deficiency animal is not particularly limited. However, out of immunocompetent cells and factors involved in immune response (specifically, B cells, T cells, HK cells, phagocyte such as macrophage, alexin, and the like), one or more cells or a part or all of factors are made in disorder by adopting a genetic technique or administering a drug, thereby producing the immune-deficiency animal. When there are many immunocompetent cells or immune response factors which are made in disorder, an immune response capability dependent thereon can be made defective, so that it is possible to further depress the rejection in transplanting the hepatic tissues in an animal. Therefore, even in case of human-derived hepatic tissues, it is possible to efficiently engraft the hepatic tissues into the immune-deficiency animal.

Examples of the immune-deficiency animal include: a nude animal whose T-cell-dependent immune response capability has been made defective; a scid animal whose T-cell-and-B-cell-dependent immune response capability has been made defective; an animal whose NK-cell-dependent immune response capability has been made defective by administering anti-asialo GM1 antibodies to the scid animal as will be described in Examples; and the like.

Note that, the anti-asialo GM1 antibodies are antibodies whose antigens are asialo GM1 proteins specifically expressed on NK cells of a mouse, and are specifically bound to the proteins so as to make the NK-cell-dependent immune response capability defective.

Further, in order to make NK-cell-involving immune response capability defective in an animal other than the mouse, a radiant ray is irradiated or a large quantity of immunosuppressant is administered.

Note that, a condition under which the sample is transplanted varies depending on which animal is to be subjected to the transplantation and which tissues the animal has, but it is possible to carry out the transplantation in accordance with a general known method. For example, in case of transplanting the hepatic tissues beneath a kidney membrane of a mouse as will be described in Examples, a mouse is anesthetized and is subjected to abdominal section, and then its kidney is exposed, and hepatic tissues are inserted beneath its kidney membrane as a sample, and a muscular coat and a skin are sutured as a single coat, thereby completing the transplantation.

Note that, in the cirrhosis model animal, whether the transplanted human hepatic tissues have been engrafted or not can be determined by HE staining for the transplanted hepatic tissues. When it is confirmed that the hepatic tissues are alive on the basis of the HE staining, it is determined that the transplanted human hepatic tissues have been engrafted. While, it is easily determined that tissues failed to be engrafted because a cell structure thereof is entirely broken. Further, as a determination method other than the foregoing method, it is possible to adopt a method of measuring proteins such as human albumin, human prealbumin, and protein C, that are synthesized in the human hepatic cells, in accordance with ELISA, Western blotting, or the like.

In the foregoing manner, the human-derived cirrhosis tissues are transplanted, thereby producing a cirrhosis model animal exhibiting the pathology of cirrhosis. The cirrhosis model animal exhibits the pathology more closely similar to the pathology of a cirrhosis patient than a conventional model animal whose hepatic tissues are damaged, so that the cirrhosis model animal can greatly contribute to development of a therapy and a therapeutic drug for cirrhosis.

In terms of medicinal properties of various materials, particularly in terms of a drug efficacy screening experiment which is carried out in order to develop a pharmaceutical agent, a disease model animal having a certain illness exhibits the pathology more closely similar to an actual clinical condition than a normal animal. Thus, it is the most preferable to carry out an experiment using not a normal animal but a disease model animal having a certain illness also in terms of evaluation of drug efficacy. Therefore, the cirrhosis model animal plays an important role as an experimental animal in clarifying a cause of a human disease, in diagnosing and preventing the disease, and in developing a therapy for the disease.

(2) Usability of the cirrhosis model animal of the present invention

The cirrhosis model animal obtained by the production method described in (1) has the following usability.
(i): According to a conventional method, developed by the Stanford University School of Medicine, in which hepatic cells are injected into a mouse from its tail (K, Marion PL, Nakai H, Meuse L, Cullen JM, Bordier BB, Schwall R, Greenberg HB, Glenn JS, Kay MA. Sustained survival of humanhepatocytes in mice: A model for in vivo infection with human hepatitis B and hepatitis delta viruses. Nat. Med. 2000 Mar; 6(3):327-331), human hepatic cells are embedded in a mouse liver in a mosaic manner, so that it is impossible to maintain the human hepatic tissues as tissues. Further, when the hepatic tissues affected with cirrhosis are dispersed and injected as hepatic cells, cells originally damaged are further damaged, so that an engraftment rate may be extremely low. In contrast, unlike the conventional method, the present invention is arranged so as to transplant the human hepatic tissues, so that the transplantation is more practical in terms of a living organism than study on drugs or various kinds of materials in the hepatic cells. As a result, it is possible to provide a clinically significant model animal.
(ii): Human-derived hepatic tissues affected with cirrhosis can be subcultured in an animal (particularly a mouse which can be easily used as an experimental animal), so that it is possible to obtain a model animal exhibiting the pathology of human cirrhosis.
(iii): It is possible to produce a cirrhosis model animal in which cirrhosis tissues are engrafted in a relatively brief period (approximately one week).
(iv): When patient-derived hepatic tissues are transplanted into a kidney of the cirrhosis model animal,
   a) it is possible to easily distinguish the kidney of the cirrhosis model animal from the transplanted tissues, and
   b) it is possible to separately transplant tissues of a right portion, a left portion, or an upper portion, a middle portion, and a lower portion of the kidney of a single organism. Thus, it is possible to carry out comparison in terms of drug efficacy under the same condition.
(v): It is possible to favorably use the production method of the present invention in: pharmaceutical agent for diagnosis of cirrhosis; a screening experiment of the pharmaceutical agent which is carried out in order to prevent and/or treat cirrhosis; and the like. This greatly facilitates development of cirrhosis therapeutic drug.
(vi): When a therapy for cirrhosis is found, it is possible to prevent cirrhosis from proceeding to hepatic cancer. This contributes to prevention of hepatic cancer.

The following Examples will further detail the present invention. Note that, the present invention is not limited to the following examples, and can be varied within a scope of claims of the present invention.

### [Example 1] (Production of cirrhosis model animal)

In the present example, scid mice each of which has T-cell and B-cell deficiency were used so as to produce cirrhosis model animals. A procedure thereof is as follows.

In the present Example, BALB mice were used as the scid mice. As pretreatment, 20 µg of anti-asialo GM1 antibodies (product of Cedarlane, type number: CL8955, product name: Ranbbit Anti-Mouse/Rat AsialoGM1 Polyclonal Antibody) were intraperitoneally administered to each of the scid mice three days before, and 20 µg of anti-asialo GM1 antibodies were intrapertioneally administered to each of the scid mice before abdominal section on the date of transplantation. Note that, for comparison, the anit-asialo GM1 antibodies were not administered to some scid mice.

Hepatic tissues extirpated from cirrhosis patient were used as human hepatic tissues affected with cirrhosis. The extirpated hepatic tissues were cut into a several-centimeter cube, and conserved in ice-cold culture.

1 cc of anesthetic containing Nembutal (product of Banyu Pharmaceutical Co., Ltd.) (4 cc of Nembutal was added to 100 cc of physiological saline) was intrapertioneally administered to each of the scid mice by using an injection syringe (2.5 cc, its needle is 27G), thereby anesthetizing the scid mice. In case where the needle of 27G is used, an enteric canal fends off the needle even when the needle is stuck vertically into an abdominal wall of the mouse, so that it is possible to make an injection without damaging the enteric canal. All limbs of the anesthetized scid mouse were engrafted with vinyl tapes, and its abdomen was subjected to midline incision by means of scissors. At this time, a skin and a fascia were separately cut open so that the enteric canal and the like were not damaged.

Subsequently, a mouse retractor was applied to the scid mouse, and the enteric canal was separated with a cotton swab or forceps, thereby exposing the kidney. At this time, effused blood was absorbed by pressing the cotton swab against the blood. Thereafter, the cotton swab was inserted behind the kidney so as to remove and roll the kidney. Further, the kidney was held with micro forceps and was slightly dissected while observing the kidney with a stereomicroscope. The hepatic tissues conserved in the culture solution were cut into an approximately 2 mm cube, and the cube of hepatic tissues was inserted beneath the membrane of the dissected kidney while holding the kidney membrane of the scid mouse with the forceps.

After the hepatic tissues were inserted in this manner, the enteric canal was returned into the abdomen. Then, the cut was stitched up by using a suture (3-0 silk). The stitch was carried out with respect to a skin and a fascia respectively. Thereafter, the scid mouse was moved to another cage, and came out from the anesthetic approximately two hours later.

### [Example 2] (Determination on whether the transplanted hepatic tissues have been engrafted or not)

A survival rate of the cirrhosis model animals produced in Example 1 was 100 %. That is, all the mice which came out from the anesthetic survived. As to the mice to which the anti-asialo GM1 antibodies had been administered before the transplantation, the anti-asialo GM1 antibodies were further administered in five days and in ten days after the transplantation.
Further, whether the human hepatic tissues had been engrafted into the kidney membrane of the mouse or not was confirmed in two weeks after the transplantation. This confirmation experiment was carried out in accordance with HE staining described in the embodiment. As a result, when the anti-asialo GM1 antibodies were not administered, a rate at which the human hepatic tissues were engrafted was 15 %. When the anti-asialo GM1 antibodies were administered, the rate at which the human hepatic tissues were engrafted was 35 %.

### [Example 3] (Comparison between the case where the anti-asialo GM1 antibodies were administered and the case where the anti-asialo GM1 antibodies were not administered in terms of the engraftment rate)

Subsequently, with respect to model animals obtained by transplanting normal human hepatic tissues in scid mice, the inventors studied differences between the case where the anti-asialo GM1 antibodies were administered and the case where the anti-asialo GM1 antibodies were not administered in the same manner as in Example 1. The differences are described as follows.

In case where the anti-asialo GM1 antibodies were not administered, the human hepatic tissues were engrafted into five mice out of nineteen model animals. In case where the anti-asialo GM1 antibodies were administered, the human hepatic tissues were engrafted into seven mice out of twelve model animals. This result shows that administration of the anti-asialo GM1 antibodies improves the rate at which the human hepatic tissues are engrafted. This may be based on the following reason: the administration of the anti-asialo GM1 antibodies causes natural-killer-cell-involving immune response in the model animal to be defective, thereby suppressing rejection dependent on the natural killer cells.

According to the foregoing result, it can be presumed that: also in case of transplanting human hepatic tissues affected with cirrhosis, administration of the anti-asialo GM1 antibodies improves the engraftment rate.

The invention being thus described, it will be obvious that the same way may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

As described above, according to the present invention, it is possible to obtain a cirrhosis model animal, having human hepatic tissues affected with cirrhosis, with a high probability. Unlike a conventional cirrhosis experimental animal, the cirrhosis model animal is produced by actually transplanting human cirrhosis tissues. Therefore, it is possible to favorably use the cirrhosis model animal in a screening experiment performed with respect to pharmaceutical agent for diagnosis of cirrhosis and a pharmaceutical agent for preventing and/or treating cirrhosis. Thus, it is expected that this technique will greatly facilitate development of cirrhosis therapeutic drug.

## Claims

1. A cirrhosis model animal, **characterized in that** a human hepatic tissue affected with cirrhosis is transplanted in a tissue of an animal.

2. The cirrhosis model animal, as set forth in claim 1, wherein the hepatic tissue is transplanted in a kidney of the animal.

3. The cirrhosis model animal as set forth in claim 1 or 2, wherein the animal is an immune-deficiency animal.

4. The cirrhosis model animal as set forth in claim 3, wherein the immune-deficiency animal is an animal whose T-cell and/or B-cell-dependent immune response capability is defective.

5. The cirrhosis model animal as set forth in claim 4, wherein the immune-deficiency animal is a nude animal or a scid animal.

6. The cirrhosis model animal as set forth in any one of claims 3 to 5, wherein the immune-deficiency animal is an animal whose natural-killer-cell-dependent immune response capability is defective.

7. The cirrhosis model animal as set forth in claim 6, wherein the natural-killer-cell-dependent immune response capability is made defective by administering an anti-asialo GM1 antibody.

8. The cirrhosis model animal as set forth in any one of claims 1 to 7, wherein the animal is a mouse.

9. The cirrhosis model animal as set forth in any one of claims 1 to 8, wherein the human hepatic tissue affected with the cirrhosis is classified as Child A in accordance with Child's classification which classifies cirrhosis in terms of severity.

10. A production method of a cirrhosis model animal, **characterized by** comprising the steps of transplanting a hepatic tissue affected with cirrhosis in an immune-deficiency-animal tissue whose immune response capability is made defective and engrafting the hepatic tissue.
